# EUROPEAN PATENT SPECIFICATION

(11) **EP 0 936 917 B1**
(45) Date of publication and mention of the grant of the patent: **12.11.2003**
(21) Application number: 97943046.9
(22) Date of filing: 19.09.1997
(51) Int. Cl.: A61K 35/20, A61P 1/04

(54) **PREVENTION OF GASTROINTESTINAL DAMAGE**
VERHÜTUNG VON MAGEN-DARM SCHÄDEN
PREVENTION DU DELABREMENT GASTRO-INTESTINAL

(30) Priority: 20.09.1996 GB 9619634
(43) Date of publication of application: 25.08.1999
(73) Proprietor: SHS International Ltd., Liverpool L7 9PT (GB)
(72) Inventor: JOHNSON, Wendy, Susan, West Kirby CH4 6DY (GB); PLAYFORD, Raymond, John, London W5 3BU (GB)
(74) Representative: Atkinson, Peter Birch
(86) International application number: GB9702572
(87) International publication number: WO98011904

(56) References cited:
- EP-A- 0 338 229
- EP-A- 0 484 148
- EP-A- 0 527 283
- WO-A-92/02538
- WO-A-93/14783
- WO-A-95/00155
- WO-A-96/34614
- CHEMICAL ABSTRACTS, vol. 111, no. 1, 3 July 1989 Columbus, Ohio, US; abstract no. 1522u, S. SUZUKI : "HUMAN EPIDERMAL GROWTH FACTOR IN BREAST MILK, EARLY NEONATAL URINE, AND AMNIOTIC FLUID: SPECULATION ON THE SOURCE AND PHYSIOLOGICAL ROLE." page 152; XP002053998 & NAGOYA MED. J., vol. 33, no. 2, 1988, pages 91-110,
- CHEMICAL ABSTRACTS, vol. 103, no. 11, 16 September 1985 Columbus, Ohio, US; abstract no. 82348b, L. JANSSON ET AL.: "MITOGENIC ACTIVITY AND EPIDERMAL GROWTH FACTOR CONTENT IN HUMAN MILK." page 121; XP002053999 & ACTA PAEDIATR. SCAND., vol. 74, no. 2, 1985, pages 250-253,
- R.J. PLAYFORD ET AL.: "BOVINE COLOSTRUM IS PROPHYLACTIC AGAINST INDOMETHACIN-INDUCED INJURY." GASTROENTEROLOGY, vol. 112, no. 4 SUPPL., April 1997, BALTIMORE, PA, US, page A394 XP002053545

## Description

The present invention relates to the prevention of gastrointestinal damage and more particularly, but not exclusively. to such damage which occurs in the intestine.

There are a variety of gastrointestinal conditions in which damage to epithelial type cells occur. For example, this damage may be in the form of ulceration, increased permeability with protein and blood loss from the intestine or structuring.

Gastrointestinal conditions involving damage to epithelial cells arise after prolonged administration of Non Steroidal Anti-inflammatory Drugs (NSAIDs) (e.g. indomethacin. ibuprofen. azapropazone. naproxen. piroxicam. ketoprofen, diclofenac. asprin etc) to patients who require protection from chronic inflammatory medical conditions. Examples of such chronic conditions which require prolonged administration of NSAIDs are rheumatoid arthritis and osteoarthritis. NSAID therapy is also beneficial for sufferers of Cystic Fibrosis (to ameliorate the inflammatory process causing lung damage). Long term use of NSAIDs is associated with a high risk of developing gastric or intestinal damage including structuring, fibrosis and ulceration. A major effect of NSAIDs is damage to gastrointestinal epithelial cells which may lead to the development of ulcers. Such ulcers may unexpectedly haemorrhage or become perforated. This leads to the requirement for emergency treatment and when undetected may even be associated with mortality. Mortality is especially associated with patients on NSAIDS who perforate because these patients are often symptomless and do not suffer pain because of the pain dampening effects of NSAIDs.

Up to 60% of NSAID-taking patients complain of dyspepsia or generalised abdominal discomfort. It has also been reported that gastric ulceration occurs in 12-30%, and duodenal lesions in 2-19%. of long term NSAID users. When it is considered that NSAIDs account for 5% of prescribed drugs in the UK but account for 25% of all reported adverse effects, it is apparent that these adverse effects associated with NSAIDs are a major problem and that a satisfactory means of preventing NSAID-induced damage to gastrointestinal epithelial cells would be of considerable benefit.

Various measures are used to treat NSAID-induced ulcers. These include the use of several types of pharmaceutical products, such as Histamine H₂ receptor antagonists, sucralfate, prostaglandins (e.g. misoprostol) and hydrogen-potassium pump inhibiting agents (e.g. omeprazole). A major disadvantage of these products is that, although they are effective at treating gastric damage, they are largely ineffective when directed towards intestinal epithelium. It is therefore desirable that there is an agent which is effective for intestinal epithelium. It is particularly desirable that there is a suitable agent that may be used prophylactically to prevent gastrointestinal damage occurring. For instance, it would be of great benefit to treat someone prophylactically if it is anticipated they will require NSAID therapy in order that gastrointestinal damage may be avoided. For example, prophylactic treatment would be of benefit for sufferers of rheumatoid arthritis, osteoarthritis or cystic fibrosis who are taking NSAIDs.

It is an object of the present invention to obviate or mitigate the above mentioned disadvantages and provide a prophylactic agent that may be used to prevent damage occurring to gastrointestinal epithelium as a result of administration of NSAIDs.

According to a first aspect of the present invention, there is provided the use of bovine colostrum or a derivative thereof for the manufacture of a medicament for prophylactic treatment of a gastrointestinal condition partially characterised by damage to epithelial cells and caused by administration of a non-steroidal anti-inflammatory drug.

By "prophylactic treatment" we mean either (i) a treatment that protects gastrointestinal epithelium such that a gastrointestinal condition that is at least partially characterised by damage to epithelial cells does not occur; or (ii) a treatment that prevents healthy epithelial cells from becoming damaged if a gastrointestinal condition already exists.

The invention is applicable for prophylactic treatment of patients who are at risk of suffering NSAID induced damage to gastrointestinal epithelial cells (such as sufferers of rheumatoid arthritis, osteoarthritis or cystic fibrosis who are taking NSAIDs). Therefore colostrum or a derivative thereof may be used to prophylactically prevent damage caused by NSAIDS such as indomethacin, ibuprofen, azapropazone, naproxen, piroxicam, ketoprofen, diclofenac or asprin.

Colostrum is the milk secreted by the mammary gland during the first 48 hours following parturition. The composition of this "first milk" is fundamentally different to that of the subsequently secreted normal milk. In particular, colostrum contains increased concentrations of growth hormones, growth factors and maternal immunoglobulins. The colostrum used in the present invention is preferably the milk obtained in the first 2 milkings following parturition. Derivatives of such milk may also be used.

The colostrum derivatives referred to in this invention are those which contain viable immunoglobulins, growth factors and /or growth hormones as may be found in colostrum. Examples of preferred colostrum derivatives are:
1. Colostrum that is spray dried to form a powder before being used. If desired the spray dried derivative may be reconstituted in the form of a spray dried skimmed milk drink. The colostrum may be defatted before spray drying if desired.
2. Colostral whey or a derivative thereof. Colostral whey is colostrum from which casein proteins have been removed. Derivatives suitable for use according to the invention include ultrafiltered or microfiltered fractions of colostral whey. These fractions contain more concentrated Growth Factors relative to remaining colostral proteins and nutrients. Colostral whey may be used in liquid form (which may be defatted if desired) or may be further treated (such as being spray dried) before use according to the invention.

The bovine colostrum may be obtained by normal milking procedures, after which it may be pooled and frozen prior to being processed, if desired, to produce a colostrum derivative.

We have found that colostrum, or a derivative thereof, has the beneficial effect of preventing gastrointestinal epithelial cells from being damaged. These beneficial effects are surprising when it is considered that in our studics normal milk, or a derivative thereof, is significantly less effective for preventing such damage.

Colostrum contains significantly higher levels of physiologically active agents such as hormones, Growth Factors and Immunoglobulins than can be found in normal milk and while we do not want to be bound by any theory, we believe this may contribute towards its efficacy in preventing the development of the abovementioned conditions. We believe these agents within colostrum, or a derivative thereof, protect epithelial cells from damage being inflicted upon them (thereby acting as a prophylactic). It is also possible that these physiologically active agents found in colostrum, or a derivative thereof, regulate apoptosis or regulate modelling of the extracellular matrix in the gastrointestinal tract.

The use of colostrum, or a derivative thereof, has many advantages over therapies currently being used for NSAID induced gastrointestinal conditions that are characterised by epithelial damage. For instance, large quantities of bovine colostrum can be readily obtained from dairy cattle. This means there are sufficient resources for the wide scale prophylactic use of colostrum to help patients who are on long term NSAID therapy. Furthermore, bovine colostrum is a natural and safe resource.

The colostrum or derivative thereof is preferably administered by an enteral route. This may be by means of an enema, an asogastric tube or alternatively by means of gastrostomy tubes or jejunostomy tubes. A most preferred route of administration is by an oral route.

It is preferred that colostrum, or a derivative thereof, is administered at least once daily. Treatment with colostrum or derivatives thereof may continue until the risk of gastrointestinal damage occurring has been removed. It is also preferred that the colostrum or a derivative thereof is administered from 6 to 72 (more preferably from 24 to 48) hours in advance of initiation of NSAID therapy and should ideally continue until NSAID therapy has stopped.

The amount of colostrum. or derivative thereof, to be administered for prophylactic treatment of an NSAID induced gastrointestinal condition that is at least partially characterised by damage to epithelial cells depends on a number of factors. These include, for example, the particular colostrum or derivative to be administered, the severity of the condition, and the age of the subject, to be treated.

We have established that, to have a protective effect on gastrointestinal epithelium, bovine colostral whey is preferably administered in the range of 30-300mls/day. 0.1-60.0 grammes/day represent a suitable daily dosage of spray dried derivatives of bovine colostrum. It will be appreciated that the amount of a colostrum derivative required will depend upon the precise extraction or purification steps undertaken to prepare such a colostrum derivative.

If desired, the colostrum (or derivative thereof) may be supplemented with one or more growth factors (e.g. purified growth factors) such as an IGF (e.g. IGF-1 or IGF-2), a transforming growth factor (e.g. TGFβ1, TGFβ2 or TGFβ3), a keratinocyte growth factor, a fibroblast growth factor, or a platelet-derived growth factor.

Colostrum, or a derivative thereof, may be formulated with other agents to form a composition suitable for enteral consumption. For instance, agents such as carbohydrates, a nitrogen source, vegetable oils, emulsifiers, oils containing long chain fatty acids, antioxidants, vitamins, soluble fibre or minerals and other trace elements may be included in the composition to fulfil nutritional requirements when colostrum is being incorporated into a food product (such as a confectionery bar) or drink product (in which case a liquid derivative or a powder derivative reconstituted as a liquid may be used). Alternatively derivatives or colostrum may be formulated with suitable excipients, stabilizers and the like to make a tablet, capsule or liquid medicament.

Additionally flavouring may also be included to make such compositions more palatable if the medicament is to be taken orally.

The invention is illustrated by the following non-limiting Examples with reference to the accompanying drawing in which:

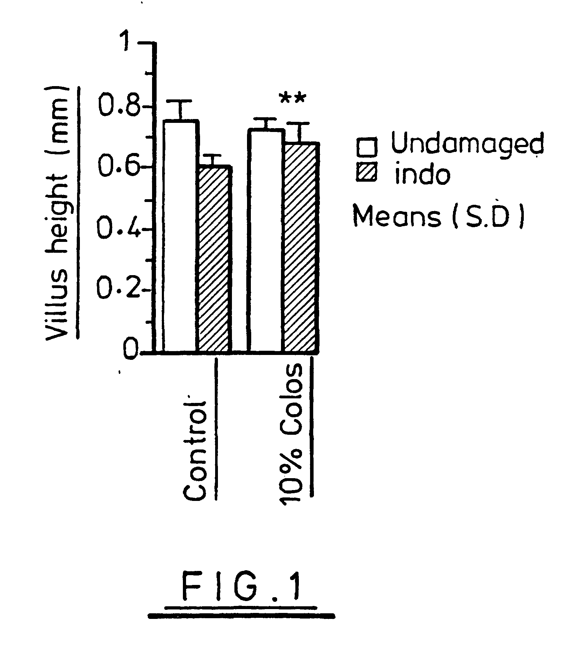

Figure 1 represents the results of Example 1 for demonstrating the effect of a colostrum derivative on indomethacin induced damage of gastrointestinal epithelium in mice.

### EXAMPLE 1

The effect of colostral whey on indomethacin-induced intestinal damage was assessed using a mouse model.

Mice were fed on a standard chow diet. A control group of mice (16/group) were given a placebo consisting of drinking water containing 10% (vol/vol) of defatted, microfiltered milk whey and a "colostrum" group was given drinking water that contained 10% (vol/vol) of defatted, microfiltered colostral whey. After two weeks on the respective diets, half the animals in each group also received indomethacin (85mg/kg subcutaneously) for a further 24 hours to induce intestinal injury. After this time the animals were sacrificed and intestinal villus height measured. Referring to Fig. 1. there was no significant difference in villus height between animals that had received colostral extract (1) and those just given placebo (2) (controls) which had not received indomethacin. Indomethacin caused significant damage (measured as a reduction in villus height) in control groups (3). However, no significant damage (** in Fig. 1) occurred in the animals that had received 10% colostral whey in their drinking water for two weeks prior to indomethacin treatment (4).

### EXAMPLE 2

### METHODS

### 1. In vivo model of small intestinal injury

### Protocol:

Mice were randomised into groups of twenty and fed on a standard chow diet *ad libitum.* The drinking water was supplemented with 10% solution of defatted colostrum or milk whey for six days. Pilot studies showed that the addition of these solutions did not affect the total volume drunk (mean 5 ml/mouse/day). Small intestinal injury was induced in half of the animals in each group by administering a single dose of indomethacin (85mg/Kg sc.). Animals were killed 24 h later. In order to assess changes in proliferation, each animal also received vincristine (1 mg/Kg i.p.) two hours prior to killing.

### Assessment of damage and proliferation:

The wet weight of the various sections of the gastrointestinal tract were recorded and samples of the small intestine and colon (defined by their percentage length) were fixed in Carnoy's fluid and stored in 70% (v/v) ethanol. Tissues were subsequently stained with the Feulgen reaction and the crypts displayed by microdissection. The numbers of arrested metaphases in 20 crypts per animal per site were counted.

Differences in villus height (as an index of intestinal damage) were determined in various regions of the intestine by microdissecting the tissue and tracing the outline of the villi using a stereo dissecting microscope. Tracings were subsequently scanned and analysed by computer image analyses.

### 2. Statistics

Studies were assessed using a two-way ANOVA (with diet and presence of indomethacin as factors). Where a significant effect was seen (P < 0.05). individual comparisons between groups were performed based on the group means, residual and degrees of freedom obtained from the ANOVA.

### RESULTS

The *in vivo* model of damage to gastrointestinal epithelium allows accurate quantitation of the degree of gaslrointestinal injury and is used to determine the protective effects of growth factors involved in mucosal integrity and repair. In these studies, maximal damage occurs 24 hours after administration of indomethacin. Colostrum treated animals have very little damage after this time. This indicates that growth factors in colostrum reduce the degree of initial damage rather than increasing the rate of repair.

Animals which received colostrum or milk solutions but had not been given indomethacin showed no significant changes in gastrointestinal epithelium proliferation or villus morphology compared to control animals.

Indomethacin caused a 25% reduction in the proliferation rate (as determined by 2-hour metaphase assessment. P <0.001) of the small and large intestine in control animals and also those receiving colostrum or milk solution.

At both jejunal and ileal sites, indomethacin also caused a 25% reduction in the villus heights of control animals. Similar changes were seen in animals which had received 10% milk solution. Animals receiving 10% colostrum, however, had only about a 5% reduction in their villus height (p < 0.001 compared to control animals receiving indomethacin). This shows that colostrum prevents damage from occurring to gastrointestinal epithelial cells.

This model demonstrates the value of colostral growth factors in preventing gastrointestinal damage. Addition of colostrum, but not milk, to the drinking water of mice markedly reduced the amount of small intestinal injury caused by indomethacin.

## Claims

1. The use of bovine colostrum or a derivative thereof for the manufacture of a medicament for prophylactic treatment of a gastrointestinal condition partially **characterised by** damage to epithelial cells and caused by administration of a non-steroidal anti-inflammatory drug.

2. The use according to claim 1 wherein the colostrum is obtained in the first 48 hours post parturition.

3. The use according to claims 1 or 2 to wherein the colostrum is obtained from the first and / or second milking post parturition.

4. The use according to any one of claims 1 to 3 wherein a colostrum derivative is used and is in a spray dried form.

5. The use according to claim 4 wherein 0.1- 60.0 grammes of the spray dried derivative is administered daily.

6. The use according to any one of claims 1 to 3 wherein a colostrum derivative is used and is a liquid reconstituted from a spray dried colostrum derivative.

7. The use according to any one of claims 1 to 3 wherein a colostrum derivative is used and that derivative is colostral whey or a colostral whey derivative.

8. The use according to claim 7 wherein the colostral whey derivative is obtained by ultrafiltration or microfiltration.

9. The use according to claims 7 or 8 wherein 30 - 300mls of colostral whey or colostral whey derivative is administered daily.

10. The use according to any preceding claim wherein a colostrum derivative is used and that derivative is defatted.

11. The use according to any preceding claim wherein colostrum or a derivative thereof is administered by an enteral route.

12. The use according to claim 11 wherein colostrum or a derivative thereof is administered by an enema, a nasogastric tube, gastrostomy tube or jejunostomy tube.

13. The use according to claim 11 or 12 wherein the colostrum, or derivative thereof, is administered in the form of a capsule or liquid.

14. The use according to claim 11 wherein colostrum or a derivative thereof is administered by an oral route.

15. The use according to claim 14 wherein the colostrum. or derivative thereof, is administered in the form of a tablet, capsule, liquid, food product or drink product.

16. The use according to claim 15 wherein the food product or drink product contains at least one of carbohydrates, a nitrogen source vegetable oils, emulsifiers, oils containing long chain fatty acids, antioxidants, vitamins, soluble fibre, minerals and flavouring.

17. The use according to any preceding claim wherein colostrum or a derivative thereof is administered, from 6 to 72 hours in advance of the non-steroidal anti-inflammatory drug.

18. The use according to claim 17 wherein colostrum or a derivative thereof is administered from 24 to 48 hours in advance of the administration of the non-steroidal anti-inflammatory drug.

19. The use according to any one of claims 1 to 18 wherein the non steroidal anti-inflammatory drug is one of indomethacin, ibuprofen, azapropazone, naproxen, piroxicam, ketoprofen, diclofenac and asprin.

## Patentansprüche

1. Verwendung von bovinem Kolostrum oder einem Derivat davon zur Herstellung eines Medikamentes zur prophylaktischen Behandlung einer gastrointestinalen Erkrankung, teilweise **gekennzeichnet durch** Schädigung der Epithelzellen und verursacht **durch** Verabreichung eines nicht-steroidalen entzündungshemmenden Arzneimittels.

2. Verwendung nach Anspruch 1, worin das Kolostrum in den ersten 48 Stunden nach der Geburt gewonnen wird.

3. Verwendung nach Anspruch 1 oder 2, worin das Kolostrum aus dem ersten und/oder zweiten Gemelk nach der Geburt gewonnen wird.

4. Verwendung nach einem der Ansprüche 1 bis 3, worin ein Kolostrum-Derivat verwendet wird und in einer sprühgetrockneten Form vorliegt.

5. Verwendung nach Anspruch 4, worin 0,1-60,0 Gramm des sprühgetrockneten Derivats täglich verabreicht werden.

6. Verwendung nach einem der Ansprüche 1 bis 3, worin ein Kolostrum-Derivat verwendet wird und eine aus einem sprühgetrockneten Kolostrum-Derivat rekonstituierte Flüssigkeit ist.

7. Verwendung nach einem der Ansprüche 1 bis 3, worin ein Kolostrum-Derivat verwendet wird und dieses Derivat Kolostrum-Molke oder ein Kolostrum-Molkenderivat ist.

8. Verwendung nach Anspruch 7, worin das Kolostrum-Molkenderivat durch Ultrafiltration oder Mikrofiltration gewonnen wird.

9. Verwendung nach Anspruch 7 oder 8, worin 30 - 300 ml Kolostrum-Molke oder Kolostrum-Molkenderivat täglich verabreicht werden.

10. Verwendung nach einem der vorangehenden Ansprüche, worin ein Kolostrum-Derivat verwendet wird und dieses Derivat entfettet ist.

11. Verwendung nach einem der vorangehenden Ansprüche, worin Kolostrum oder ein Derivat davon über eine enterale Route verabreicht wird.

12. Verwendung nach Anspruch 11, worin Kolostrum oder ein Derivat davon über einen Einlauf, eine Nasensonde, Gastrostomiesonde oder Jejunostomiesonde verabreicht wird.

13. Verwendung nach Anspruch 11 oder 12, worin das Kolostrum oder Derivat davon in der Form einer Kapsel oder Flüssigkeit verabreicht wird.

14. Verwendung nach Anspruch 11, worin Kolostrum oder ein Derivat davon über ein orale Route verabreicht wird.

15. Verwendung nach Anspruch 14, worin das Kolostrum oder Derivat davon in der Form einer Tablette, Kapsel, Flüssigkeit, eines Nahrungsmittel- oder Getränkeprodukts verabreicht wird.

16. Verwendung nach Anspruch 15, worin das Nahrungsmittel- oder Getränkeprodukt mindestens eines von Kohlenhydraten, einer Stickstoffquelle, pflanzlichen Ölen, Emulgatoren, Ölen mit langkettigen Fettsäuren, Antioxidanzien, Vitaminen, löslichen Fasern, Mineralen und Geschmacksstoffen enthält.

17. Verwendung nach einem der vorangehenden Ansprüche, worin Kolostrum oder ein Derivat davon von 6 bis 72 Stunden vor dem nicht-steroidalen entzündungshemmenden Arzneimittel verabreicht wird.

18. Verwendung nach Anspruch 17, worin Kolostrum oder ein Derivat davon von 24 bis 48 Stunden vor der Verabreichung des nicht-steroidalen entzündungshemmenden Arzneimittel verabreicht wird.

19. Verwendung nach einem der Ansprüche 1 bis 18, worin das nicht-steroidale entzündungshemmende Arzneimittel eines von Indometacin, Ibuprofen, Azapropazon, Naproxen, Piroxicam, Ketoprofen, Diclofenac und Aspirin ist.

## Revendications

1. L'utilisation de colostrum bovin ou d'un dérivé de celui-ci dans la fabrication d'un médicament utilisé dans le traitement prophylactique d'une pathologie gastro-intestinale **caractérisée** en partie par un endommagement des cellules épithéliales et causée par l'administration d'un anti-inflammatoire non stéroïdien.

2. L'utilisation selon la Revendication 1, dans laquelle le colostrum est obtenu dans les 48 heures qui suivent la parturition.

3. L'utilisation selon la Revendication 1 ou 2, dans laquelle le colostrum est obtenu à la première et/ou la seconde traite après la parturition.

4. L'utilisation selon l'une quelconque des Revendications 1 à 3, dans laquelle un dérivé du colostrum est employé sous une forme déshydratée pulvérisée.

5. L'utilisation selon la Revendication 4, dans laquelle le dérivé déshydraté pulvérisé est administré quotidiennement à raison de 0,10-60,0 grammes par jour.

6. L'utilisation selon l'une quelconque des Revendications 1 à 3, dans laquelle un dérivé du colostrum est employé, qui est sous une forme liquide obtenue par reconstitution à partir d'un dérivé du colostrum déshydraté et pulvérisé.

7. L'utilisation selon l'une quelconque des Revendications 1 à 3, dans laquelle un dérivé du colostrum est employé, ledit dérivé correspondant au lactosérum colostral ou à un dérivé du lactosérum colostral.

8. L'utilisation selon la Revendication 7, dans laquelle le dérivé du lactosérum colostral est obtenu par ultrafiltration ou microfiltration.

9. L'utilisation selon la Revendication 7 ou 8, dans laquelle le lactosérum colostral ou le dérivé du lactosérum colostral est administré quotidiennement à raison de 30-300 ml par jour.

10. L'utilisation selon l'une quelconque des Revendications précédentes, dans laquelle un dérivé du colostrum est employé et ce dérivé dégraissé.

11. L'utilisation selon l'une quelconque des Revendications précédentes, dans laquelle le colostrum ou un dérivé du colostrum est administré par voie entérale.

12. L'utilisation selon la Revendication 11, dans laquelle le colostrum ou un dérivé du colostrum est administré au moyen d'un lavement médicamenteux, d'une sonde nasogastrique, d'une sonde de gastrostomie ou d'une sonde de jéjunostomie.

13. L'utilisation selon la Revendication 11 ou 12, dans laquelle le colostrum ou un dérivé du colostrum est administré sous la forme d'une gélule ou d'un liquide.

14. L'utilisation selon la Revendication 11, dans laquelle le colostrum ou un dérivé du colostrum est administré par voie orale.

15. L'utilisation selon la Revendication 14, dans laquelle le colostrum ou un dérivé du colostrum est administré sous la forme d'un comprimé, d'une gélule, d'un liquide, d'un produit alimentaire ou d'un produit buvable.

16. L'utilisation selon la Revendication 15, dans laquelle le produit alimentaire ou le produit buvable contient un au moins des composants suivants: hydrates de carbone, source d'azote, huiles végétales, émulsifiants, huiles contenant des acides gras à chaîne longue, anti-oxydants, vitamines, fibres solubles, éléments minéraux et aromatisant.

17. L'utilisation selon l'une quelconque des Revendications précédentes, dans laquelle le colostrum ou un dérivé du colostrum est administré 6 à 72 heures avant l'anti-inflammatoire non stéroïdien.

18. L'utilisation selon la Revendication 17, dans laquelle le colostrum ou un dérivé du colostrum est administré 24 à 48 heures avant l'administration d'un anti-inflammatoire non stéroïdien.

19. L'utilisation selon l'une quelconque des Revendications 1 à 18, dans laquelle l'anti-inflammatoire non stéroïdien est l'un des suivants: indométacine, ibuprofène, azapropazone, naproxène, piroxicam, kétoprofene, diclofénac et aspirine.
